Europäisches Patentamt

European Patent Office

Office européen des brevets

(19)

(11) Veröffentlichungsnummer: **0 111 735**
**B1**

(12) **EUROPÄISCHE PATENTSCHRIFT**

(45) Veröffentlichungstag der Patentschrift:
06.04.88

(51) Int. Cl.⁴: **A 61 F 9/00**

(21) Anmeldenummer: **83111258.6**

(22) Anmeldetag: **11.11.83**

(54) **Verfahren zur Gewinnung von Tränenflüssigkeit.**

(30) Priorität: **19.11.82 DE 3243339**

(43) Veröffentlichungstag der Anmeldung:
**27.06.84 Patentblatt 84/26**

(45) Bekanntmachung des Hinweises auf die Patenterteilung:
**06.04.88 Patentblatt 88/14**

(84) Benannte Vertragsstaaten:
**AT BE CH DE FR GB IT LI LU NL SE**

(56) Entgegenhaltungen:
**DE-A-2 261 549**
**FR-A-2 308 941**

(73) Patentinhaber: **Opel, Helmut, Dr.med.,
Hudtwalckerstrasse 2-8, D-2000 Hamburg 60 (DE)**

(72) Erfinder: **Opel, Helmut, Dr.med.,
Hudtwalckerstrasse 2-8, D-2000 Hamburg 60 (DE)**

(74) Vertreter: **Schulmeyer, Karl- Heinz, Dr., Kieler
Strasse 59a, D-2087 Hasloh (DE)**

**Beschreibung**

Vorliegende Erfindung betrifft ein Verfahren zur Gewinnung von Tränenflüssigkeit mit Hilfe von Kontaktlinsen.

Die Kenntnis der Zusammensetzung der Tränenflüssigkeit, und zwar der möglichst unveränderten Tränenflüssigkeit, wie sie bei der Ruhesekretion auf der Hornhaut des Auges vorliegt, ist nur durch entsprechende analytische Untersuchungen an dem Auge entnommener Tränenflüssigkeit zu erhalten. Diese Kenntnis ist in der Praxis z.B. wichtig für die Frage, ob einem Kunden geraten werden kann, Kontaktlinsen zu tragen, oder für die Frage, wie es um die Verträglichkeit verschiedener Kontaktlinsentypen in bezug auf einen bestimmten Kunden steht. Es läßt sich nämlich nicht verhindern, daß die aufgesetzte Kontaktlinse die normale Tränenzirkulation auf der Hornhautoberfläche beeinflußt. Da die Tränenflüssigkeit u.a. als Träger von Nähr- und Wirkstoffen dient, die sie an die Hornhaut heranführt, kann eine Störung der Zirkulation der Tränenflüssigkeit auch zu einer unerwünschten Beeinträchtigung des Metabolismus der Hornhaut mit entsprechenden nachteiligen Folgeerscheinungen führen. Aufgrund der charakteristischen Zusammensetzung der Tränenflüssigkeit kann der erfahrene Augenarzt bzw. Augenoptiker aus den verschiedenen Typen von Kontaktlinsen solche auswählen, die voraussichtlich für den Träger verträglich sind. Ferner können bei Kunden, die bereits seit längerer Zeit Kontaktlinsen tragen, aufgrund der Zusammensetzung der Tränenflüssigkeit ggf. Rückschlüsse auf eine drohende oder eingetretene Störung des Metabolismus an der Hornhaut gezogen werden.

Um solche Untersuchungen durchführen zu können, benötigt man ein möglichst einfaches, aber wirkungsvolles Verfahren zur Gewinnung von Tränenflüssigkeit. Die bisher bekannten Verfahren zur Entnahme von Tränenflüssigkeit sind praktisch nicht ohne eine Reizung des Auges durchführbar. So werden beispielsweise beim Schirmer-Test zum Nachweis der Tränensekretionsmenge schmale Fließpapierstreifen auf die Hornhaut bzw. in die untere Übergangsflate des Auges gelegt. Die untersuchte Person empfindet diese Prozedur nicht als angenehm, da sich beim Auflegen der Fließpapierstreifen ein leichtes Brennen auf dem Auge nicht vermeiden läßt. Es kommt infolge der unvermeidlichen Augenreizung zu einer erhöhten Produktion der Tränenflüssigkeit, deren Zusammensetzung sich jedoch von dem normalen, sogenannten Ruhesekret deutlich unterscheidet, da der Hauptteil der zusätzlich abgesonderten Tränenflüssigkeit durch eine stärkere Anregung der Hauptränendrüse erzeugt wird, wobei der Anteil der wässrigen Komponente ansteigt und gleichzeitig eine Änderung der Tonizität und des pH-Wertes in basischer Richtung eintritt.

Auch die Verwendung einer Pipette zum Ansaugen von Tränenflüssigkeit aus dem inneren Lidwinkel kann selbst bei sehr geschickter Anwendung nicht ohne eine Augenreizung durchgeführt werden. Auch in diesem Falle tritt daher eine unerwünschte vermehrte Absonderung von Tränenflüssigkeit auf mit den damit zwangsweise verbundenen Änderungen in der Zusammensetzung der Tränenflüssigkeit.

Auch bei anderen Versuchen, die Produktion der Tränenflüssigkeit anzuregen, beispielsweise durch eine vorsichtige Massage des Auges oder durch die Anwendung gewisser Reize, beispielsweise des Reizes, der von frisch geschälten Zwiebeln ausgeht, werden die oben erwähnten Nachteile nicht vermieden, ganz abgesehen davon, daß solche Behandlungen für den Probanden nicht gerade angenehm sind.

Mit Hilfe dieser bekannten Verfahren kann man daher nur Tränenflüssigkeiten erhalten, die infolge der Augenreizung einen Überschuß an der wäßrigen Komponente enthalten, deren Zusammensetzung sich demnach deutlich von der einer unverdünnten Tränenflüssigkeit, d.h. eines Ruhesekrets, unterscheidet. Mit den bekannten Verfahren ist es daher nicht möglich, reine Ruhesekrete zu gewinnen, mit denen die unverfälschte Zusammensetzung der Tränenflüssigkeit ermittelt werden kann.

Der vorliegenden Erfindung liegt daher die Aufgabe zugrunde, ein Verfahren zu schaffen, bei dem die bei den bekannten Verfahren zur Gewinnung von Tränenflüssigkeit auftretenden Nachteile vermieden werden und das darüberhinaus erlaubt, eine merklich größere Ausbeute an Tränenflüssigkeit in einem einzigen Arbeitsgang zu erreichen.

Diese Aufgabe wird erfindungsgemäß gelöst durch ein Verfahren zur Gewinnung von Tränenflüssigkeit, bei dem man eine weiche hydrophile Kontaktlinse mit einem Wassergehalt von 38 bis 85 % mit Tränenflüssigkeit in Kontakt bringt, wobei die Kontaktlinse Tränenflüssigkeit aufnimmt, und das dadurch gekennzeichnet ist, daß die die Tränenflüssigkeit enthaltende Kontaktlinse mit einer Extraktionslösung, die 0,8 bis 4 % Salze enthält, von denen mindestens ein Teil Natriumchlorid ist, bei einer Temperatur von 20° bis 24°C 30 Minuten bis 2 Stunden lang behandelt wird, wobei die Tränenflüssigkeit aus der Kontaktlinse extrahiert wird.

Es war überraschend, als festgestellt wurde, daß die an sich bekannten weichen hydrophilen Kontaktlinsen nicht nur in der Lage sind, in relativ kurzer Zeit eine beachtliche Menge an Tränenflüssigkeit einschließlich der darin gelösten Substanzen aufzunehmen, sondern daß sie diese Tränenflüssigkeit in relativ kurzer Zeit wieder an geeignete Extraktionslösungen abgeben. Da die Kontaktlinsen praktisch keine nennenswerten Augenreizungen hervorrufen, kommt es auch nicht zu einer erhöhten Produktion der Hauptränendrüse an einem mehr wässrigen, aber proteinarmen Sekret und dem damit verbundenen unerwünschten, weil verfälschenden Verdünnungseffekt. Es ist vielmehr möglich, durch die vom Probanden als sehr schonend empfundene Anwendung der Kontaktlinse die Tränenflüssigkeit in unverfälschter Zusammensetzung zu erhalten Das erfindungsgemäße Verfahren schafft damit die Voraussetzungen für die Ermittlung der genauen Zusammensetzung der Tränenflüssigkeit in Form eines Ruhesekrets.

Die in dem erfindungsgemäßen Verfahren eingesetzten weichen hydrophilen Kontaktlinsen sind an sich bekannt und im Handel erhältlich. Diese Kontaktlinsen zeichnen sich durch einen hohen Wassergehalt von 30 bis 85 % sowie durch eine besondere Weichheit des Materials aus. Die Aufnahmekapazität der hydrophilen Kontaktlinsen an Tränenflüssigkeit steigt im allgemeinen mit ihrem Wassergehalt, weshalb hydrophile Kontaktlinsen mit einem Wassergehalt von 65 bis 85% besonders gut geeignet sind. Ferner hängt die Brauchbarkeit der hydrophilen Kontaktlinsen für das erfindungsgemäße Verfahren von der Art und Zusammensetzung der verwendeten Copolymeren ab. Als vorteilhaft haben sich hydrophile Kontaktlinsen aus Methylmethacrylat/Vinyl-pyrrolidon-Copolymeren (abgekürzt: MMA/VP-Copolymer) erwiesen, z.B. solche im Verhältnis MMA/VP von 30 : 70 mit einem Wassergehalt von 70 %. Besonders geeignet sind hydrophile Kontaktlinsen aus 2-Hydroxyethylmethacrylat/Vinylpyrrolidon-Copolymeren (abgekürzt: HEMA/VP-Copolymer), da sie eine außerordentlich hohe Aufnahmekapazität für Tränenflüssigkeiten aufweisen. Beispielsweise sind Copolymere aus HEMA/VP im Gewichtsverhältnis 50 : 50 mit einem Wassergehalt von 72 %, oder mit einem Gewichtsverhältnis von 60 : 40 und einem Wassergehalt von 68 % oder mit einem Gewichtsverhältnis von 10 : 90 und einem Wassergehalt von 85 % besonders gut geeignet.

Die Kontaktlinsen haben üblicherweise ein Gewicht von etwa 50 bis 70 mg.

Die Kontaktlinse wird zur Aufnahme der Tränenflüssigkeit nur relativ kurze Zeit vom Probanden getragen, da sich die Einstellung des Gleichgewichts zwischen der Menge an aufgenommener und wieder abgegebener Tränenflüssigkeit in der Kontaktlinse (die sogenannte "Äquilibrierung") in der Regel innerhalb von 30 Minuten bis 2 Stunden vollzogen hat.

Nachdem die Äquilibrierung der Kontaktlinse mit der Tränenflüssigkeit erreicht ist, u. U. jedoch auch früher, wird die Kontaktlinse vom Auge entfernt und, ggf. nach kurzem Abtrocknen mit Fließpapier, in eine geeignete Extraktionslösung eingelegt, die 0,8 bis 4 % Salze enthält, von denen mindestens ein Teil Natriumchlorid ist. Die Extraktionslösung wird auf einer Temperatur von 20° bis 24°C gehalten. Zur Beschleunigung der Extraktion wird die Lösung gerührt oder geschüttelt, so daß die Extraktion in der Regel nach 30 Minuten, in schwierigeren Fällen nach 2 Stunden, beendet ist.

Die von der Tränenflüssigkeit befreite Kontaktlinse wird danach aus der Lösung genommen und die Lösung anschließend für die analytischen Untersuchungen zur Festellung der in der Tränenflüssigkeit befindlichen Substanzen und Wirkstoffe verwendet. Die analytischen Untersuchungen sind an sich bekannt und bedienen sich in der Regel mikroanalytischer Verfahren, z. B. der Biuret-Reaktion zur Bestimmung des Proteingehaltes in der Tränenflüssigkeit, oder der selektiven Lysozymbestimmung nach Selsted und Martinez.

Der Erfolg des erfindungsgemäßen Verfahrens hängt außer von der Qualität der eingesetzten weichen hydrophilen Kontaktlinse insbesondere auch von der Art und Zusammensetzung der Extraktionslösung ab. In einer besonders einfachen, bevorzugten Ausführungsform des erfindungsgemäßen Verfahrens enthält die Extraktionslösung 0,85 % Natriumchlorid, womit sie eine Isotonizität besitzt, die ungefähr derjenigen der Tränenflüssigkeit entspricht. Eine solche Extraktionslösung wird bevorzugt eingesetzt, da sie für die Kontaktlinsen besonders zuträglich ist und nicht zu befürchten ist, daß auch bei länger andauernder Behandlung eine Quellung oder Schrumpfung der Kontaktlinse verursacht wird. Eine solche Extraktionslösung ist vorzugs weise neutral, d. h. der pH-Wert liegt bei etwa 7.

Es kann sich in manchen Fällen jedoch auch als zweckmäßig erweisen, zur Verbesserung der Extraktion der Tränenflüssigkeit bzw. bestimmter in ihr enthaltener Substanzen aus der Kontaktlinse den pH-Wert zu erhöhen oder zu erniedrigen. Daher wird in einer weiteren bevorzugten Ausführungsform des erfindungsgemäßen Verfahrens eine Extraktionslösung eingesetzt, deren pH-Wert mit einer Base, wie Natriumhydroxid oder Ammoniumhydroxid, bis auf 8 bis 11 erhöht worden ist. Andererseits kann in einer weiteren bevorzugten Ausführungsform auch eine Extraktionslösung eingesetzt werden, deren pH-Wert mit Hilfe einer verdünnten flüssigen Säure, wie z. B. Essigsäure, Propionsäure usw., auf bis zu 6 bis 2,5 erniedrigt worden ist.

Um insbesondere eine möglichst vollständige und rasche Extraktion vorzugsweise der Proteinbestandteile der Tränenflüssigkeit zu erreichen, kann es ferner von Vorteil sein, die Polarität der Extraktionslösung zu ändern, z. B. dadurch, daß man vorzugsweise eine Extraktionslösung mit einem Gehalt von 1 bis 10 % Dioxan oder ebenfalls bevorzugt mit einem Gehalt von 5 bis 50 % Ethylenglykol einsetzt.

Ferner kann es von Vorteil sein, wenn die eingesetzte Extraktionslösung zur wirksameren Extraktion von Proteinen sogenannte "Wasserstoffbrückensprenger" enthalten. Als solche können vorzugsweise Harnstoff oder Guanidin-Hydrochlorid beigefügt werden, wobei man ersteren vorteilhaft in Form einer achtmolaren Lösung, letztere als sechsmolare Lösung einsetzt.

Schließlich können auch solche Salze zu der Extraktionslösung zugefügt werden, die die hydrophobe Wechselwirkung zwischen Kontaktlinse und Protein herabsetzen und damit ebenfalls die Extraktion der Proteine erleichtern. Hierzu gehören z. B. Natriumrhodanid-, Trichloressigsäure- oder Natriumjodidlösungen, z. B. in dreimolarer Lösung.

In jedem Fall soll jedoch der Gesamtsalzgehalt der Extraktionslösung 4 Gew.-% nicht überschreiten.

Ein Vorteil der vorliegenden Erfindung besteht darin, daß im Unterschied zu den bisher bekannten und angewandten Verfahren zur Gewinnung von Tränenflüssigkeit das erfindungsgemäße Verfahren eine in der Regel reizlose Gewinnung von Tränenflüssigkeit aus dem Auge ermöglicht. Dadurch wird die durch Reizung am Auge verursachte erhöhte Produktion von zusätzlicher Tränenflüssigkeit aus der Haupttränendrüse, die im Vergleich zu dem Ruhesekret einen Überschuß an wässriger Komponente enthält und eine Verschiebung des pH-Wertes in basischer Richtung sowie eine Herabsetzung der Tonizität zeigt, vermieden. Die erfindungsgemäß gewonnene Tränenflüssigkeit entspricht daher bezüglich der Eigenschaften und Zusammensetzung weitgehend der normalen Tränenflüssigkeit, d. h. dem sogenannten Ruhesekret. Mit Hilfe der vorliegenden Erfindung können daher wesentliche Fehlerquellen bei der Bestimmung der Zusammensetzung der normalen Tränenflüssigkeit eliminiert werden.

Ein weiterer Vorteil der Erfindung besteht darin, daß größere Mengen an normaler Tränenflüssigkeit gewonnen werden können, als dies mit den bekannten Verfahren möglich ist. Beispielsweise können durch Pipettieren nur ca. 5 bis 6 µl Tränenflüssigkeit gewonnen werden, und dies nur unter Reizung des Auges, d. h. durch stärkere Anregung der Haupttränendrüse, mit den dadurch eintretenden nachteiligen Folgen. Hingegen kann mit dem erfindungsgemäßen Verfahren unter Verwendung einer hydrophilen Kontaktlinse, z. B. einer solchen aus HEMA/VP-Copolymeren, hergestellt aus 60 % 2-Hydroxyethylmethacrylat und 40 % N-Vinylpyrrolidon, mit einem Wassergehalt von 65 %, die ein Gewicht von ca. 50 mg aufweist, eine Menge von ca. 25 µl Tränenflüssigkeit aufgenommen werden, d. h. eine fünfmal größere Menge, als durch Pipettieren erhalten werden kann. Eine solche Menge läßt sich natürlich bei der Mikroanalyse leichter handhaben und erlaubt sicherere analytische Ergebnisse. Mit anderen Kontaktlinsen, nämlich solchen aus HEMA/VP-Copolymeren mit einem Wassergehalt von ca. 70 bis 85 %, lassen sich noch weit größere Mengen an Tränenflüssigkeit in einem Arbeitsgang gewinnen.

Die Erfindung wird anhand eines Beispiels näher erläutert.

**Beispiel**

Eine Kontaktlinse aus HEMA/VP-Copolymerisat, hergestellt aus 60 % HEMA und 40 % VP, mit einem Wassergehalt von 65 % wurde auf das linke Auge eines Probanden gebracht. Während der 30 Minuten, die die Kontaktlinse auf dem Auge blieb, wurde von dem Probanden keine merkliche Augenreizung registriert. Es wurde auch keine über die normale Menge hinausgehende Menge an Tränenfluß beobachtet. 30 Minuten nach dem Einsetzen der Kontaktlinse wurde diese wieder herausgenommen, kurz mit Filterpapier getrocknet und in 0,2 ml einer 0,85 %-igen Natriumchloridlösung, die auf 21°C temperiert war, eingelegt. Die Extraktion der Tränenflüssigkeit aus der Kontaktlinse in die Extraktionslösung erfolgte unter 30 Minuten langem Schütteln der Lösung. Danach wurde die Kontaktlinse aus der Extraktionslösung entfernt.

Die die Tränenflüssigkeit enthaltende Extraktionslösung wurde anschließend auf den Gehalt der Tränenflüssigkeit an Gesamtprotein geprüft, und zwar mit Hilfe der Biuret-Reaktion. Das Biuret-Reagenz bestand aus 6,0 g Kalium-natriumtartrat, gelöst in 300 ml einer 2,5 n Natriumhydroxydlösung, zu der 1,5 g $CuSo_4.5 H_2O$ und 1 g Kaliumjodid zugefügt wurden. Diese Lösung wurde mit kohlendioxidfreiem Wasser auf 1 Liter aufgefüllt.

0,8 ml des Biuret-Reagenzes wurden zu der Extraktionslösung zugefügt und die Mischung bei Raumtemperatur stehengelassen. Nach 30 Minuten erfolgte die Vermessung der Lösung im Photometer bei 540 nm gegen einen Blindwert. Die Messung kann auch durch visuellen Farbvergleich vorgenommen werden.

Die Auswertung erfolgte über eine vorher angefertigte Eichkurve. Diese war wie folgt aufgestellt worden: Kontaktlinsen aus HEMA/VP-Copolymerisat (60 % HEMA, 40 % VP) mit einem Wassergehalt von 65 % wurden in 0,85 %-igen Natriumchloridlösungen, die gelöstes Albumin in abgestuften Konzentrationen von 0,1 bis 1,5 % enthielten, jeweils 30 Minuten lang geschüttelt, wobei die Kontaktlinsen eine gewisse Menge der eiweißhaltigen Lösung aufnahmen, die in etwa der Gleichgewichtsmenge entsprachen. Danach erfolgte die Extraktion der jeweiligen Kontaktlinse in 0,2 ml einer 0,85 %-igen Natriumchloridlösung während 30 Minuten unter Schütteln. Dann wurde die Kontaktlinse aus der Lösung entfernt und die Menge an Albumin in der Lösung mit Hilfe der Biuret-Reaktion photometrisch bei 540 nm bestimmt. Die gemessenen Extinktionswerte wurden gegen die Konzentration des Albumins graphisch aufgetragen.

Aus der so erhaltenen Eichkurve ergab sich ein Gesamtgehalt an Protein in der untersuchten Tränenflüssigkeit von 0,75 % (w/v).

Die visuelle Auswertung wurde in der Weise ausgeführt, daß zum Vergleich eine Kontaktlinse der gleichen Art und mit gleicher Dimension wie diejenige, die für die Untersuchung eingesetzt wurde, mit einer 0,7 %-igen Albuminlösung in der gleichen Weise, wie vorstehend beschrieben, behandelt wurde. Die 0,7 %-ige Albuminlösung entspricht in etwa der Gesamtproteinkonzentration, die man in der Regel in einer "normalen" Tränenflüssigkeit, d. h. in dem Ruhesekret, findet. Nach Entfernung der Kontaktlinsen und Durchführung der Biuret-Reaktion in den erhaltenen Extraktionslösungen wurde die Farbintensität durch visuellen Farbvergleich festgestellt. Das Ergebnis stimmte mit dem der Extinktionsmessung gut überein.

Anstelle des Gesamtproteingehaltes kann beispielsweise aus der die extrahierte Tränenflüssigkeit enthaltenden Extraktionslösung auch der Gehalt der Tränenflüssigkeit an Lysozym bestimmt werden. Lysozym ist ein wichtiges Ferment, das in der Lage ist, die Bakterienflora in der Tränenflüssigkeit zu kontrollieren und gefährliche Keime abzutöten. Es ist daher insbesondere bei Personen, die Kontaktlinsen tragen wollen, zum Zwecke der Beratung wichtig, die Beschaffenheit der Tränenflüssigkeit vor allem hinsichtlich des Lysozymgehaltes zu kennen.

Die Bestimmung des Lysozymgehalts erfolgt in der die extrahierte Tränenflüssigkeit enthaltenden Extraktionslösung selektiv nach der Methode von M.E. Selsted und R.J. Martinez, Analytical Biochemistry 109, Seiten 67-70 (1980). Dabei wird als Substrat eine Suspension von desaktivierten Zellen von Micrococcus lysodeikticus verwendet.

Im Prinzip beruht diese Bestimmungsmethode darauf, daß eine Kontaktlinse, die Tränenflüssigkeit enthält, in der vorstehend angegebenen Weise extrahiert wird, und zwar in einer Extraktionslösung, die zusätzlich eine

4

Suspension von Micrococcuszellen der angegebenen Art enthält. Diese Extraktionslösung ist infolge der Suspension stark trübe. Nach erfolgter Extraktion wird je nach der Menge an Lysozym, die in der Tränenflüssigkeit enthalten ist, die Trübung mehr oder weniger stark reduziert. Diese Änderung hat eine Änderung der Extinktion zur Folge, so daß die Lysozymkonzentration auf diese Weise durch photometrische Messungen bei 540 nm ermittelt werden kann.

Entsprechende Versuche wurden mit 16 verschiedenen Probanden durchgeführt. Die Ergebnisse dieser Untersuchungen sind in der folgenden Tabelle zusammengestellt. Man erkennt unschwer, daß sich die verschiedenen Kontaktlinsenmaterialien auch recht unterschiedlich bezüglich der Aufnahmekapazität der Tränenflüssigkeit bzw. des Lysozymgehaltes verhalten. Besonders zufriedenstellend ist die Aufnahmekapazität von Kontaktlinsen aus HEMA/VP-Copolymeren, und auch Kontaktlinsen aus MMA/VP-Copolymeren bringen zufriedenstellende Ergebnisse. Dagegen ist das harte Kontaktlinsenmaterial Celluloseacetobutyrat für die Gewinnung von Tränenflüssigkeit ungeeignet.

**Tabelle**

Bestimmung des Lysozymgehaltes in aus Kontaktlinsen extrahierten Tränenflüssigkeiten

| Versuch Nr. | Kontaktlinsen-material | Lysozymgehalt der Kontaktlinsen (KL) ($\mu$g/mg feuchte KI) | ($\mu$g/KL) | Gewicht der Kontaktlinsen (mg) |
|---|---|---|---|---|
| 1 | MMA/VP+) | 0,24 | 6,7 | 27,57 |
| 2 | MMA/VP | 0,32 | 11,6 | 36,79 |
| 3 | MMA/VP | 0,56 | 26,1 | 46,79 |
| 4 | MMA/VP | 0,55 | 24,5 | 44,93 |
| 5 | MMA/VP | 0,30 | 11,5 | 37,91 |
| 6 | HEMA/VP++) | 38,96 | 1909,1 | 49,00 |
| 7 | HEMA/VP | 49,11 | 2312,0 | 47,08 |
| 8 | HENA/VP | 45,59 | 1885,5 | 41,35 |
| 9 | HEMA/VP | 7,87 | 475,3 | 60,38 |
| 10 | HEMA/VP | 8,64 | 475,4 | 55,00 |
| 11 | HEMA/VP | 13,28 | 925,7 | 69,65 |
| 12 | HEMA/VP | 9,85 | 675,2 | 68,56 |
| 13 | HEMA/VP | 12,83 | 850,2 | 66,27 |
| 14 | HEMA/VP | 6,09 | 325,3 | 53,37 |
| 15 | PHEMA/+++) | 0,18 | 6,6 | 37,39 |
| 16 | CAB ++++) | 0,09 | 1,6 | 18,35 |

+) MMA/VP = Methylmethacrylat/Vinylpyrrolidon-Copolymer (ca. 70 % $H_2O$)
++) HEMA/VP = 2-Hydroxyethylmethacrylat/Vinylpyrrolidon-Copolymer (ca. 70 % $H_2O$)
+++) PHEMA = Poly-2-hydroxyethylmethacrylat (ca. 40 % $H_2O$)
++++) CAB = Celluloseacetobutyrat (= hartes Kontaktlinsenmaterial)

**Patentansprüche**

1.Verfahren zur Gewinnung von Tränenflüssigkeit, bei dem man eine weiche hydrophile Kontaktlinse mit einem Wassergehalt von 38 bis 85 % mit Tränenflüssigkeit in Kontakt bringt, wobei die Kontaktlinse Tränenflüssigkeit aufnimmt, dadurch gekennzeichnet, daß die die Tränenflüssigkeit enthaltende Kontaktlinse mit einer Extraktionslösung, die 0,8 bis 4 % Salze enthält, von denen mindestens ein Teil Natriumchlorid ist, bei einer Temperatur von 20° bis 24°C 30 Minuten bis 2 Stunden lang behandelt wird, so daß die Tränenflüssigkeit aus der Kontaktlinse extrahiert wird.

2. Verfahren nach Anspruch 1, dadurch gekennzeichnet, daß eine Extraktionslösung mit einem pH-Wert von etwa 7 eingesetzt wird.

3. Verfahren nach Anspruch 1, dadurch gekennzeichnet, daß eine Extraktionslösung eingesetzt wird, deren pH-Wert mit einer Base bis auf etwa 8 bis 11 eingestellt ist.

4. Verfahren nach Anspruch 1, dadurch gekennzeichnet, daß eine Extraktionslösung eingesetzt wird, deren pH-Wert mit einer verdünnten flüchtigen Säure bis auf 6 bis 2,5 eingestellt ist.

5. Verfahren nach einem der Ansprüche 1 bis 4, dadurch gekennzeichnet, daß eine Extraktionslösung mit einem Gehalt von 0,85 % Natriumchlorid eingesetzt wird.

6. Verfahren nach einem der Ansprüche 1 bis 5, dadurch gekennzeichnet, daß eine Extraktionslösung mit einem Gehalt von 1 bis 10 % Dioxan eingesetzt wird.

7. Verfahren nach einem der Ansprüche 1 bis 5, dadurch gekennzeichnet, daß eine Extraktionslösung mit einem Gehalt von 5 bis 50 % Ethylenglykol eingesetzt wird.

8. Verfahren nach einem der Ansprüche 1 bis 7, dadurch gekennzeichnet, daß eine Extraktionslösung mit einem Gehalt an Harnstoff oder Guanidin-Hydrochlorid eingesetzt wird.

## Claims

1. Method of collecting tear fluid, in which a soft hydrophilic contact lens with a water content of 38 to 85 % is brought into contact with tear fluid, whereby the contact lens absorbs some tear fluid, characterised in that the contact lens containing the tear fluid is treated for 2 hours at a temperature of 20° to 24° with an extraction solution which contains 0.8 to 4 % of salt, of which at least one part is sodium chloride, so that the tear fluid is extracted from the contact lens.

2. Method according to Claim 1, characterised in that an extraction solution with a pH-value of about 7 is used.

3. Method according to Claim 1, characterised in that an extraction solution is used, the pH-value of which is adjusted with a base to about 8 to 11.

4. Method according to Claim 1, characterised in that an extraction solution is used, the pH-value of which is adjusted with a dilute volatile acid to 6 to 2.5.

6. Method according to one of the Claims 1 to 5, characterised in that an extraction solution with a 1 to 10 % dioxan content is used.

7. Method according to one of the Claims 1 to 5, characterised in that an extraction solution with a 5 to 50 % ethylene glycol content is used.

8. Method according to one of the Claims 1 to 7, characterised in that an extraction solution with a urea or guanidine-hydrochloride content is used.

## Revendications

1. Procédé pour recueillir du fluide lacrymal, dans lequel on met en contact un verre de contact hydrophile souple avec une teneur en eau comprise entre 38 et 85 % avec du liquide lacrymal, qui est absorbé par le verre de contact, caractérisé en ce que le verre de contact, contenant du liquide lacrymal, est placé pendant 30 minutes à 2 heures dans une solution d'extraction, contenant entre 0,8 et 4 % de sels, dont une partie au moins est du chlorure de sodium, se trouvant à une température comprise entre 20 et 24°C, pour extraire le liquide lacrymal du verre de contact.

2. Procédé suivant la revendication 1, caractérisé en ce que l'on utilise une solution d'extraction, dont le pH est réglé au voisinage de 7.

3. Procédé suivant la revendication 1, caractérisé en ce que l'on utilise une solution d'extraction, dont le pH est réglé à l'aide d'une base entre environ 8 et 11.

4. Procédé suivant la revendication 1, caractérisé en ce que l'on utilise une solution d'extraction, dont le pH est ajusté à l'aide d'un acide volatil dilué entre 6 et 2,5.

5. Procédé suivant l'une des revendications 1 à 4, caractérisé en ce que l'on utilise une solution d'extraction avec une teneur en chlorure de sodium de 0,85 %.

6. Procédé suivant l'une des revendications 1 à 5, caractérisé en ce que l'on utilise une solution d'extraction avec une teneur en dioxanne comprise entre 1 et 10 %.

7. Procédé suivant l'une des revendications 1 à 5, caractérisé en ce que l'on utilise une solution d'extraction avec une teneur en éthylène-glycol comprise entre 5 et 50 %.

8. Procédé suivant l'une des revendications 1 à 7, caractérisé en ce que l'on utilise une solution d'extraction contenant de l'urée ou du chlorhydrate de guanidine.